# EUROPEAN PATENT SPECIFICATION

(11) **EP 2 288 333 B1**
(45) Date of publication and mention of the grant of the patent: **28.12.2011**
(21) Application number: 09747513.1
(22) Date of filing: 13.05.2009
(51) Int. Cl.: A61K 8/365, A61Q 5/00

(54) **PROSTAGLANDIN BASED COMPOSITIONS AND METHOD OF USE THEREOF**
AUF PROSTAGLANDIN BASIERENDE ZUSAMMENSETZUNG UND VERFAHREN ZU IHRER VERWENDUNG
COMPOSITIONS À BASE DE PROSTAGLANDINE ET SON PROCÉDÉ D'UTILISATION

(30) Priority: 14.05.2008 US 127686
(43) Date of publication of application: 02.03.2011
(73) Proprietor: Peter Thomas Roth Labs, Llc, New York, NY 10022 (US)
(72) Inventor: ROTH, Peter, Thomas, New York NY 10022 (US); BLAKE, Bridget, New York NY 10022 (US)
(74) Representative: Haile, Alison Victoria
(86) International application number: PCT/US2009/043850
(87) International publication number: WO 2009/140430

(56) References cited:
- WO-A1-03/066008
- WO-A2-01/74307
- US-B1- 6 262 105
- M. WIESE ET AL.: "Interaction of Prostaglandin E1 with alpha-Cyclodextrin in Aqueous Systems: Stability of the Inclusion Complex" JOURNAL OF PHARMACEUTICAL SCIENCES, vol. 80, no. 2, February 1991 (1991-02), pages 153-156, XP002542045 cited in the application
- Anonymous: "17-phenyl trinor Prostaglandin E2 serinol amide Catalog N° 10004238" Cayman Chemical, Ann Arbour, USA, Cayman Chemical Product Information XP002542046 Retrieved from the Internet: URL:http://www.caymanchem.com/pdfs/1000423 8.pdf> [retrieved on 2009-08-19]

## Description

### Field of the Disclosure

The disclosure relates to a process and composition for enhancing the appearance of one or more properties associated with hair follicles in a subject, such as luster, sheen, brilliance, gloss, glow, shine, patina, length, thickness, density, or overall appearance of the hair using a prostaglandin based compound, such as a prostaglandin or prostamide alone or in association with a stabilizing agent, and/or with a topical cosmetic carrier.

### Background of the Disclosure

Prostaglandin based compounds, such as prostaglandins, prostamides, as well as their derivative and analogs, have been reported to be useful for enhancing the appearance of hair. However, many prostaglandin based compounds are unstable, and this instability can undermine their effectiveness. For instance, prostaglandin E is unstable in aqueous environments and decomposes to Prostaglandin A and Prostaglandin B.

### Summary of the Disclosure

The present disclosure is based, in part, upon the discovery that specific prostaglandin based compounds are relatively more stable in aqueous environments than others; and further that by complexing a prostaglandin based compound of the disclosure with a stabilizing agent, such as cyclodextrin, the prostaglandin may be stabilized and provided in a cosmetic composition that can be used to enhance the appearance of hair, such as the hair of the eyelashes, eye-brows, and head.

In certain aspects, the disclosure is directed to a topical composition, such as a composition that is intended for application to mammalian skin. For instance, in certain embodiments, a composition of the disclosure is formulated so as to be applied to skin and or hair comprising the eyebrow or eyelash. The composition may include one or more of an effective amount of a prostaglandin based compound; a stabilizing agent; and optionally, a biologically acceptable carrier. In some embodiments, the prostaglandin based compound includes the following structure: 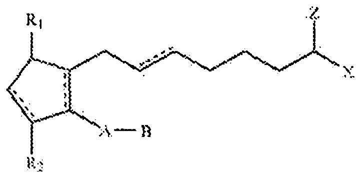 wherein the dashed bonds represent a single or double bond which can be in the cis or trans configuration;

A is an alkylene or alkenylene radical having from two to six carbon atoms, which radical may be interrupted by one or more oxa radicals and substituted with one or more hydroxy, oxo, alkyloxy or akylcarboxy groups wherein said alkyl radical comprises from one to six carbon atoms;

B is a cycloalkyl radical having from three to seven carbon atoms, or an aryl radical, selected from the group consisting of hydrocarbyl aryl and heteroaryl radicals having from four to ten carbon atoms wherein the heteroatom is selected from the group consisting of nitrogen, oxygen and sulfur atoms;
X is either -N(H)CH(CH₂OH)₂ or -OCH(CH₂OH)₂;
Z is =O;
one of R₁ and R₂ is =O, -OH or a -O(CO)R₃ group, and the other one is -OH or -O(CO)R₃, or R₁ is =O and R₂ is H, wherein R₃ is a saturated or unsaturated acyclic hydrocarbon group having from 1 to 20 carbon atoms, or -(CH₂)mR₄ wherein m is 0 or an integer of from 1 to 10, and R₄ is cycloalkyl radical, having from three to seven carbon atoms, or a hydrocarbyl aryl or heteroaryl radical, as defined above, or a pharmacologically acceptable acid addition salt thereof.

In certain aspects, the disclosure is directed to a method of using a topical composition such as that disclosed herein for the purpose of enhancing the appearance of one or more properties associated with hair follicles in a subject, such as luster, sheen, brilliance, gloss, glow, shine, patina, length, thickness, density, or overall appearance or the hair. In such an instance, a composition of the disclosure may be contacted with the hair and or skin, e.g., which skin may be associated with the hair of a user, in such a manner that the active agent(s) of the composition is capable of penetrating into the skin. In certain embodiments, a method of the disclosure includes contacting the skin and/or hair of the eyelids, eyelashes, eyebrows, crown, arms, legs, etc. with a composition of the disclosure.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

Other features and advantages of the invention will be apparent from the following detailed description and claims.

### Detailed Description of the Invention

Prostaglandins are lipid compounds derived from cyclic fatty acids, e.g., prostanoic acid. Prostaglandins and prostamides are structurally related small molecules that modulate cellular activities through various cell surface receptors. Typically, both prostaglandins and prostamides contain at least a 20 carbon atom backbone, which backbone includes a 5-carbon (cyclopentane) ring. There are several main types of prostaglandins (PG): PG A, PG B, PGC, PGD, PGE, PGF, PGH, and PGI, based on the structure of the cyclopentane ring. The naturally occurring prostaglandins are types E, F, A, and B, with PGA and PGB being oxidation products of PGE and PGF.

Many cells of the body have specific receptors for PG types E and F, and thus, there are a large number of cells of the body that are responsive to PGE and F, whereas the number of cells of the body that have receptors responsive to PGA and/or PGB are comparatively much lower. Accordingly, PGE and PGF are much more active than their derivatives PGA and PGB.

Prostamides differ from prostaglandins in that with respect to prostaglandins the 1-position comprises a carboxylic acid, and with respect to prostamides, the 1-position comprises an amide group. Further, it has been determined that prostamides exert their effects through receptors that are distinct from prostaglandins, and thus, are biologically distinct there from. See: "Prostamide Antagonist," Woodward et al., Dept. of Biological Sciences, Allergan, Inc.

Prostaglandins or prostamides, derivatives and analogs thereof, are known to produce a variety of effects. For example, prostaglandin analogs of PGF2α and PGE2 are known to effectuate at least the appearance of hair growth in mammals. See, for instance, Sasaki et al., Exp. Dermatol. (2005) May; 14(5):323-8 and Colombe et al., Exp. Dermatol. (2007) Sep; 16(9):762-9. In fact, US Patent No. 6,262,105 to Johnstone is directed to the use of a prostaglandin F compound, and US Patent No. 7,351,404 is directed to the use of various particular prostamide compounds, the use of which compounds in cosmetic formulations purportedly stimulates hair growth.

However, prostaglandins and prostamides, as well as the derivatives and analogs thereof, are unstable in aqueous environments. For instance, in aqueous environments, PGEs and PGFs readily decompose into their less active PGA and PGB derivatives. Specifically, the E and F type prostaglandins possess a hydroxyl group at C11 of the cyclopentene ring, which in the presence of water is readily dehydrated, thereby forming a double bond within the pentene ring indicative of the PGA and PGB types. Accordingly, in order to maximize the effectiveness of such compounds in cosmetic compositions for increasing the appearance of hair growth, a prostaglandin/prostamide composition should be formulated in such a manner so as to prevent or at least reduce the decomposition of the particular prostaglandin and/or prostamide employed as the active ingredient of the cosmetic composition.

To prevent the rapid decomposition of prostaglandins, prostamides, their derivatives and analogs, the use of various stabilizers has been proposed, which stabilizers when formulated with various prostaglandins purportedly function to protect the prostaglandin from decomposition. For instance, various prostaglandins have been formulated in conjunction with cyclodextrin so as to increase the stability of the prostaglandin in solution. See, for instance, Gonzales, et al. J. Clin. Pharmacol. (2007) Jan;47(1):121-6, as well as Wiese et al., J. Pharm. Sciences 80:153-156 (1991); Szejtli, J., "Industrial Applications of Cyclodextrins," Inclusion Compounds III, Academic Press, London, England (1984), pp. 355-368.

Specifically, it was postulated that a portion of the prostaglandin was capable of being inserted within the hydrophobic cavity of the cyclodextrin so as to form an inclusion product therewith. See, for instance, Gu, et al. Yao Xue Xue Bao, (2004) Sep;39(9):742-6. However, despite the combination of prostaglandins with stabilizers, such as cyclodextrin, the decomposition of prostaglandin based active agents in solution remains problematic. Without being bound to theory, it is believed with respect to cyclodextrin, that due in part to the long, stretched out structure of the typical prostaglandin like backbone and also due in part to the hydrophilic nature of the carboxyl group at the 1-position, prostaglandins, generally, and some prostamides, are not particularly well suited for forming an inclusion product with cyclodextrins.

Accordingly, as will be described in greater detail herein below, the present inventors have determined that the prostaglandin based compounds of the present disclosure are more stable in aqueous environments, and in instances wherein the prostaglandin based compounds are combined with stabilizer, such as cyclodextrin, are more suited for forming inclusion products therewith than other such compounds heretofore known and used in cosmetic formulations. Additionally, the present inventors have determined that the prostaglandin based compounds of the present disclosure are capable of increasing the appearance of one or more hair properties such as luster, sheen, brilliance, gloss, glow, shine, patina, length, thickness, density, and/or overall appearance. Therefore, the prostaglandin based compounds of the disclosure, are useful in cosmetic compositions, particularly wherein the cosmetic composition includes one or more of a stabilizer and an aqueous vehicle, which compositions are useful in a method for the enhancement of the appearance one or more hair properties such as luster, sheen, brilliance, gloss, glow, shine, patina, length, thickness, density, and/or overall appearance.

Consequently, in certain aspects, the present disclosure is directed to tropical compositions that include at least one prostaglandin based compound, which compound may, in certain instances, be formulated in conjunction with a stabilizer. In certain aspects, a topical composition of the disclosure is provided for the conditioning and/or enhancing the look of the skin and/or hair, for instance, the skin comprising the hair of the eyelashes, eyebrows, and scalp, e.g., of a mammal, such as a human. Accordingly, a composition including a prostaglandin based compound of the disclosure will be set out first, followed by the description of the use of such a composition in a cosmetic formulation for increasing the appearance of one or more hair properties, such as luster, sheen, brilliance, gloss, glow, shine, patina, length, thickness, density, and/or overall appearance.

In one aspect, the disclosure provides a composition containing a prostaglandin based compound, which composition may be used for increasing the appearance of one or more hair properties such as luster, sheen, brilliance, gloss, glow, shine, patina, length, thickness, density, and/or overall appearance. Specifically, a prostaglandin based compound of the disclosure may include a prostaglandin or prostamide, and may optionally include a stabilizing agent. The composition may additionally include a solubilizer, an uptake and/or distribution booster, a thickening agent, a conditioning agent, a moisturizing agent, an anti-oxidant, a surfactant, and/or the like. Hence, in some embodiments, the active ingredients, e.g., a prostaglandin or prostamidemay be provided as a pre-blend in which a prostaglandin based compound is mixed first with a solublizer, such as a C₁₂₋₁₅ alkyl benzoate. Any suitable solvent may also be used, e.g., lower alcohols such as isopropanol and/or propylene glycol. Further, a stabilizing agent may also be included. In certain embodiments, a stabilizing agent may be a cyclodextrin, e.g., 2-hydroxypropyl-alpha (or beta)-cyclodextrin, sulfobutyl ether-beta-cyclodextrin, and 2,6-dimethyl-beta cyclodextrin.

The compositions may further include a cosmetically acceptable vehicle, such as a vehicle that acts as a dilutant, dispersant, and/or carrier for the prostaglandin based compound, so as to facilitate its distribution and uptake when the composition is applied to the skin, hair, and/or scalp. The cosmetically acceptable vehicle may vary from, for instance, 2%-5% to 99.9%, for instance, from 25% to 80%, including 35% to 60% by weight of the composition, and can, in the absence of other cosmetic adjuncts, form the balance of the composition.

A prostaglandin or prostamide, in the composition of the disclosure includes a prostaglandin based compound with the following formula: 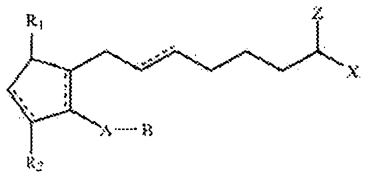 wherein the dashed bonds represent a single or double bond which can be in the cis or trans configuration,

A is an alkylene or alkenylene radical having from two to six carbon atoms, which radical may be interrupted by one or more oxa radicals and substituted with one or more hydroxy, oxo, alkyloxy or akylcarboxy groups wherein said alkyl radical comprises from one to six carbon atoms;

B is a cycloalkyl radical having from three to seven carbon atoms, or an aryl radical, selected from the group consisting of hydrocarbyl aryl and heteroaryl radicals having from four to ten carbon atoms wherein the heteroatom is selected from the group consisting of nitrogen, oxygen and sulfur atoms;
X is either -N(H)CH(CH₂OH)₂ or -OCH(CH₂OH)₂;
Z is =O;
one of R₁ and R₂ is =O, -OH or a -O(CO)R₃ group, and the other one is -OH or -O(CO)R₃, or R₁ is =O and R₂ is H, wherein R₃ is a saturated or unsaturated acyclic hydrocarbon group having from 1 to 20 carbon atoms, or -(CH₂)mR₄ wherein m is 0 or an integer of from 1 to 10, and R₄ is cycloalkyl radical, having from three to seven carbon atoms, or a hydrocarbyl aryl or heteroaryl radical, as defined above or a pharmacologically acceptable acid addition salt thereof.

In certain embodiments, the prostaglandin based compound is a prostaglandin such as [(2-hydroxy-1-hydroxymethyl)ethyl]-9-oxo-11,15-dihydroxy-17-phenyl-18, 19, 20-trinor-prosta-5Z, 13E-dien-1-oate. For instance, in certain embodiments, the prostaglandin based compound of the disclosure comprises the following structure:

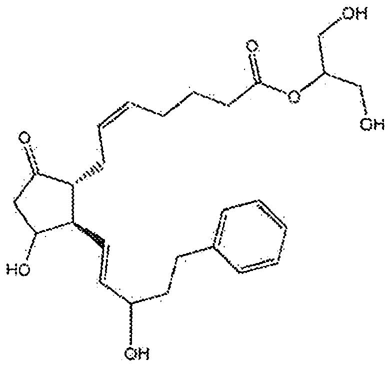

In certain embodiments, the prostaglandin based compound is a prostamide, such as 17-phenyl trinor prostaglandin E2 serinol amide. For instance, in certain embodiments, the prostaglandin based compound of the disclosure comprises the following structure:

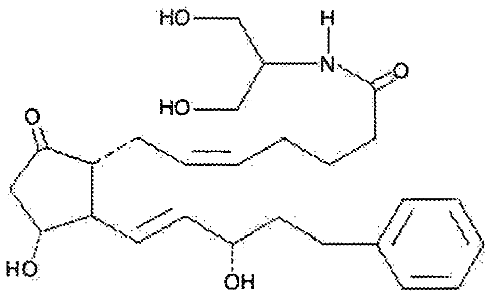

As set forth above, the present inventors have postulated that the representative prostaglandin based compounds of the present disclosure are more stable in aqueous environments, and in instances wherein the prostaglandin based compounds are combined with a stabilizer, such as cyclodextrin, are more suited for forming inclusion products therewith than other such compounds heretofore known and used in cosmetic formulations. For instance, without being held to theory, the present inventors have postulated that given the long, strung out structure of the prostaglandin like compounds of the disclosure, and in particular given the chemical nature of the X group, e.g., the - N(H)CH(CH₂OH)₂, the Applicants believe that a compound of the disclosure is capable of folding back on itself and forming an intermolecular bond with one or more of the R₁ and R₂ of the pentimer ring. For example, it is believed that in solution the diol of the X group may form an intermolecular bond (either through deprotonation and/or hydrogen bonding) with the oxygen containing groups of the pentimer ring, thereby causing the molecule to fold over on itself. It is believed that as a result of this "folding over" the pentimer ring will be better protected from hydolitic cleavage, and therefore, will be more stable in aqueous solutions, and thus less likely to decompose. Additionally, it is further believed that this "folding over" will allow the molecule to more readily fit within the ring structure of a stabilizing agent such as cyclodextrin.

Specifically, without wishing to be bound by theory, it is postulated that combining a prostaglandin based compound of the disclosure, such as 17-phenyl trinor prostaglandin E2 serinol amide, with a stabilizing agent, such as cyclodextrin, increases the stability and effectiveness of the prostaglandin composition. For instance, as indicated above, cyclodextrin has a toroid or ring-like structure that allows various portions of the prostaglandin based compound of the disclosure to be fit within the cavity of the ring structure so as to form a protective shell around the prostaglandin based compound, which protective shell may protect the pentimer ring from decomposer due to hydrolysis while in solution. For example, because the compound is both folded over and fitted within the cyclodextrin ring, water molecules in the solution will have less access to the reactive R₁ and R₂ groups of the pentimer ring, which consequently may result in less hydrolysis and therefore greater stability in solution.

The cyclodextrin thus may serve at least three main functions, 1) it promotes the solubility of the prostaglandin based compound and thus allows for a greater concentration of the compound to be present in the solution, 2) it protects various reactive groups on the prostaglandin based compound from hydrolysis, thus preventing degradation, and 3) it helps with penetration.

For example, cyclodextrins form toriod structures, which structures include an outer-surface and an inner-surface, wherein the inner surface defines a cavity. Because of the configuration of the toroid structure, the outer surface thereof is comprised of primary and secondary hydroxyl groups, which makes the outer surface relatively hydrophilic. While the outer surface is comprised of hydroxyl groups, the inner surface remains relatively less hydrophilic, and thus, capable of receiving a portion of the hydrophobic prostaglandin based compound of the disclosure within the cavity of the cyclodextrin, which cavity is less hydrophilic than the aqueous environment of the composition. Accordingly, with respect to promoting the solubility of the prostaglandin based compound, without being held to theory, it is postulated that the cyclodextrin molecules suitable for use as stabilizing agents in accordance with the disclosure are capable of forming inclusion products with the prostaglandin based compounds disclosed herein, thereby modifying the compound's typical physical and chemical properties, thus, rendering the naturally hydrophobic prostaglandin based compounds more soluble in aqueous environments.

Further, with respect to the protection of various reactive groups on the prostaglandin based compound from hydrolysis, with out being held to theory, it is postulated that the cyclodextrin molecules suitable for use as stabilizing agents in accordance with the disclosure are capable of receiving at least a portion of the cyclopentene ring of the prostaglandin based compounds described herein within the cavity of the toroid structure of the cyclodextrin, thereby protecting any associated =OH and or -OH groups positioned thereon, e.g., at C₉ or C₁₁, from hydrolysis. For instance, it is postulated that, as described in greater detail above, the prostaglandin based compounds of the disclosure will be capable of forming intramolecular bonds between the terminal isopropyl diol group and one or more of the -OH groups positioned on the cyclopentene ring, e.g., at C₁₁. This folded structure may then be inserted into the cavity of the cyclodextrin and thereby be protected from degradation due to dehydration.

Additionally, with respect to promoting penetration, with out being held to theory, it is postulated that the cyclodextrin molecules suitable for use as stabilizing agents in accordance with the disclosure are capable of acting as a permeation enhancer, for instance, by decreasing the barrier function of lipophilic membranes, such as the lipohilic membrane of the skin. Topical administration of prostaglandin based compounds is problematic because of the barrier to penetration that the stratum corneum, e.g., skin, presents. The skin is comprised of layers, which layers secrete a slightly acidic oily film which functions as a permeation barrier to water and other hydrophilic compositions. Specifically, the skin of the face has a high buffer capacity that actively resists changes in pH.

Accordingly, in order to be effective, a prostaglandin based compound contained within a cosmetic composition of the disclosure must be able to penetrate through the outer, or epidermis, layers of the skin and into the deeper layers of the dermis without greatly affecting the overall pH of the skin. Cyclodextrin helps facilitate the penetration of the prostaglandin based compound in part because it reduces the effect of the composition on the pH barrier of the skin. Hence, a prostaglandin based compound, when complexes with cyclodextrin, or other stabilizing agent, is able to form a synergistic combination that promotes the penetration of the prostaglandin based compound through the epidermis and into the dermal regions of the skin without substantially disrupting the pH barrier of the skin. Specifically, without being held to theory, it is postulated that the cyclodextrin forms an inclusion complex with the prostaglandin based compound, wherein the cyclodextrin acts as permeation enhancer carrying the prostaglandin based compound through the aqueous barrier of the cosmetic solution, from the bulk solution towards the lipophilic surface of epidermis and into the dermis, wherein the prostaglandin based compound partitions from the complex and into the dermis.

Any suitable cyclic oligosaccharide may be used as a stabilizing agent in combination with a prostaglandin based compound of the disclosure. A suitable cyclic oligosaccharide may be an oligosaccharide composed of 5 or more α-D-glucopyranosides. For instance, in certain embodiments, the cyclic oligosaccharide may be a cyclodextrin, such as an α-, β- (e.g., Cavamax W7®), γ-cyclodextrin, or the like. For example, the cyclodextrin may be a 2-hydroxypropyl-alpha-cyclodextrin, 2-hydroxypropyl-beta-cyclodextrin, sulfobutyl ether-beta-cyclodextrin, 2,6-dimethyl-beta cyclodextrin, or other alkylcyclodextrin, such as methylcyclodextrin, and the like. Other suitable agents capable of forming complexes with the prostaglandin based compounds of the disclosure may also be used as a stabilizing agent. For instance, a suitable stabilizing agent may include dextran, dextrin, pullulan, derivatives and/or analogs thereof.

Accordingly, in one aspect a composition of the disclosure includes one or more of a prostaglandin based compound thereof in a synergistic combination with one or more stabilizers (as described above), solubilizers, buffering agents or cosmetically acceptable vehicles.. For instance, in various embodiments, a suitable buffering agent may be NaOH, Ammonia, Citric Acid, KOH, Benzoic Acid, TEA, AMP, or the like. Specifically, the prostaglandin based active ingredients may be provided as a pre-blend in which a prostaglandin based compound of the disclosure is mixed with a solublizer, such as a C₁₂₋₁₅ alkyl benzoate, or may be provided as a direct blend, e.g., not provided prior in a pre-blend such as with C₁₂₋₁₅ alkyl benzoate. A suitable solvent may also be included, e.g., a lower alcohol, such as isopropanol and/or propylene glycol.

The cosmetic composition may also include a cosmetically acceptable vehicle, which vehicle may act as a dilutant, dispersant, or carrier for the prostaglandin based compound, so as to facilitate its distribution and uptake when the composition is applied to the skin, hair, and/or scalp. The cosmetically acceptable vehicle may function, in part, to facilitate the distribution and uptake of the prostaglandin based compound when the composition is applied to the skin and/or hair or scalp. Cosmetically acceptable vehicles may include water, and/or other liquid or solid emollients, solvents, humectants, thickeners and powders.

A cosmetically acceptable vehicle may comprise from 2%-5% or less to 99.9% or more, such as from 25% to 80%, including 40% to 60% by weight of the composition. Vehicles other than or in addition to water can include liquid or solid emollients, solvents, humectants, thickeners and powders. The cosmetic composition may comprise an aqueous, aqueous/alcoholic or oily solution; dispersion of the lotion type; anhydrous or lipophilic gel; emulsions of liquid or semi-liquid consistency, which may be obtained by dispersion of a fatty phase in an aqueous phase (O/W) or conversely (W/O); or may comprise a suspension or emulsion of smooth, semi-solid or solid consistency of the cream or gel type.

Accordingly, a cosmetic composition of the disclosure may be formulated as a lotion, which lotion may be prepared so as to be applied to the skin, and may comprise a liquid or semiliquid preparation in which solid particles, including the prostaglandin based compound of the disclsoure, are present in a lipid, alcohol or water base. A suitable lotion of the disclosure may be a suspension of solids, and may include a liquid oily emulsion of the oil-in-water type. A suitable lotion may also include a suspending agent, so as to produce a better dispersion, as well as a compound useful for localizing and holding the prostaglandin based compound in contact with the skin, e.g., methylcellulose, sodium carboxymethyl-cellulose, or the like.

A suitable solution may comprise a homogeneous mixture prepared by dissolving one or more chemical substances (solute) in another liquid such that the molecules of the dissolved substance may be dispersed among those of the solvent. The solution may contain other cosmetically acceptable chemicals to buffer, stabilize or preserve the solute as described herein. Suitable solvents may include ethanol, water, propylene glycol or any other cosmetically acceptable vehicle known in the art..

When a composition of the disclosure is formulated as an oily solution or gel, the fatty phase may constitute more than 90% of the total weight of the composition. When the composition is formulated as an emulsion, the proportion of the fatty phase may range from 5% to 80% by weight, and may range from 10% to 50% by weight, relative to the total weight of the composition. Oils, emulsifiers and co-emulsifiers incorporated in the composition in emulsion form may be selected from among those known in the art. The emulsifer and coemulsifier may be present in the composition at a proportion ranging from 0.03%-3% or less to 30% or more by weight, for instance, from 0.5% to 20% by weight, relative to the total weight of the composition.

Suitable oils that may be used include mineral oils (liquid petrolatum), plant oils (liquid fraction of karite butter, sunflower oil), animal oils (perhydrosqualen(e), synthetic oils (purcellin oil), silicone oils (cyclomethicone) and fluoro oils (perfluoropolyethers). Fatty alcohols, fatty acids (stearic acid) and waxes (paraffin wax, carnauba wax and beeswax) may also be used as fats. Emulsifiers that may be used include glycerol stearate, polysorbate 60, PEG-6/PEG-32/glycol stearate mixture, etc. Solvents that may be used include the lower alcohols, such as ethanol, isopropanol, and propylene glycol.

An oil or oily material may be present, together with an emollient to provide either a water-in-oil emulsion or an oil-in-water emulsion, depending largely on the average hydrophilic-lipophilic balance (HLB) of the emollient employed. Levels of such emollients may range from 0.5% to 50%, preferably between 5% and 30% by weight of the total composition. Emollients may be classified under such general chemical categories as esters, fatty acids and alcohol, polyols and hydrocarbons.

Esters may be mono- or di-esters. Acceptable examples of fatty di-esters include dibutyl adipate, diethyl sebacate, diisopropyl dimerate, and dioctyl succinate. Suitable straight chain fatty esters include lauryl palmitate, myristyl lactate, oleyl eurcate and stearyl oleate. Preferred esters include coco-caprylate/caprate (a blend of coco-caprylate and coco-caprate), propylene glycol myristyl ether acetate, diisopropyl adipate and cetyl octanoate. Suitable branched chain fatty esters include 2-ethyl-hexyl myristate, isopropyl stearate and isostearyl palmitate. Suitable tribasic acid esters include triisopropyl trilinoleate and trilauryl citrate.

Suitable fatty alcohols and acids include those compounds having from 10 to 20 carbon atoms, such as cetyl, myristyl, palmitic and stearyl alcohols and acids. Among the polyols which may serve as emollients are linear and branched chain alkyl polyhydroxyl compounds. For example, propylene glycol, sorbitol and glycerin are preferred. Polymeric polyol, such as polypropylene glycol and polyethylene glycol, may also be used. Butylene and propylene glycol may be used as penetration enhancers.

Exemplary hydrocarbons which may serve as emollients are those having hydrocarbon chains anywhere from 12 to 30 carbon atoms, such as, mineral oil, petroleum jelly, squalene and isoparaffins.

The compositions of the invention may contain a surfactant in an aqueous base. The surfactants can be present, alone or in a mixture, and may be contained in an amount from 1 to 50 % by weight, for instance, from 2.5 to 30 % by weight, including 2-5% to 20% by weight. Nonionic surfactants, amphoteric surfactants, zwitterionic surfactants and anionic surfactants may also be used.

Suitable anionic surfactants include, e.g. alkaline or alkaline earth salts, alpha-olefin sulfonates, sulfosuccinates, disodium laureth-3 sulfosuccinate, disodium PEG-5 lauryl citrate sulfosuccinate, disodium ricinolamido MEA-sulfosuccinate or disodium laurylamido MEA-sulfosuccinate and alkyl ether carboxylates.

Suitable nonionic surfactants include e.g. alkoxylated fatty alcohols, alkoxylated fatty acid esters, alkoxylated partial glycerides, saturated or unsaturated fatty acids, alkoxylated polyol esters, and alkylpolyglucosides, such as coconut glucosides, lauryl glycosides or decylglucosides. For example, ethoxylated lauryl alcohol, tetradecyl alcohol, cetyl alcohol, oleyl alcohol or stearyl alcohol, which are used alone or in mixtures with each other, as well as fatty alcohols of ethoxylated lanolin, are suitable as fatty alcohol ethoxylates. Furthermore the ethoxylated fatty acid sugar esters known as nonionic surfactants, especially ethoxylated sorbitan fatty acid ester, are suitable for use in the cosmetic preparations according to the invention. The suitable ethoxylated fatty acid sugar esters include those marketed under the trade names Tween™ and Arlacel™ by ICI surfactants and the alkyl-polyglycosides, which are marketed under the trade names Plantaren™ or Plantacare™ by Henkel or under the trade name Oramix™ by Seppic.

Suitable hydrophobic (water-insoluble) surfactants, include, for example, an emulsifying wax, polyoxyethylene acid, polyoxyethylene alcohol, glycerol monostearate, sorbitan tristearate, sorbitan monopalmitate, sorbitan sesquiloleate, and other sorbitan fatty acid ester. Anionic surfactants, nonionic surfactants, amphoteric surfactants, and zwitterionic surfactants may also be used. For instance, suitable anionic surfactant include, e.g. alkaline or alkaline earth salts, alpha-olefin sulfonates, sulfosuccinates, disodium laureth-3 sulfosuccinate, disodium PEG-5 lauryl citrate sulfosuccinate, disodium ricinolamido MEA-sulfosuccinate or disodium laurylamido MEA-sulfosuccinate and alkyl ether carboxylates. Suitable nonionic surfactants may include e.g. alkoxylated fatty alcohols, alkoxylated fatty acid esters, alkoxylated partial glycerides, saturated or unsaturated fatty acids, alkoxylated polyol esters, and alkylpolyglucosides, such as coconut glucosides, lauryl glycosides or decylglucosides. For example, ethoxylated lauryl alcohol, tetradecyl alcohol, cetyl alcohol, oleyl alcohol or stearyl alcohol, which are used alone or in mixtures with each other, as well as fatty alcohols of ethoxylated lanolin, are suitable as fatty alcohol ethoxylates. Furthermore the ethoxylated fatty acid sugar esters known as nonionic surfactants, especially ethoxylated sorbitan fatty acid ester, are suitable for use in the cosmetic preparations according to the invention. The suitable ethoxylated fatty acid sugar esters include those marketed under the trade names Tween™ and Arlacel™ by ICI surfactants and the alkyl-polyglycosides, which are marketed under the trade names Plantaren™ or Plantacare™ by Henkel or under the trade name Oramix™ by Seppic. Suitable amphoteric surfactants may include for example betaines, such as cocoamidopropylbetaine or lauryl betaine, sulfobetaines, such as cocoamidopropyl hydroxysultaine, glycinates, such as cocoamphoglycinate (INCI-name: sodium cocoamphoacetate) and diglycinates and propionates, such as cocoampho-propionate. A surfactant may be present, alone or in a mixture, and may be contained in an amount that ranges from 1% or less to 50 % or more by weight, especially preferably from 1 to 30 % by weight.

The compositions of the disclosure may also include additives and adjuvants, such as hydrophilic or lipophilic gelling agents, hydrophilic or lipophilic active agents, preservatives, antioxidants, solvents, fragrances, fillers, bactericides, odor absorbers and dyestuffs or colorants. The amounts of these various additives and adjuvants may range from 0.01% or less to 10% or more of the total weight of the composition. Depending on their nature, these additives and adjuvants may be introduced into the fatty phase or into the aqueous phase.

A suitable thickener may also be included. A thickener may be present in an amount that ranges from 0.01% or less to 20% or more by weight, such as from 0.5%-1% to 10% by weight of the composition. Suitable thickeners may be cross-linked polyacrylate materials, such as Carbopol. Gums may also be employed, such as xanthan, carrageenan, gelatin, karaya, pectin or locust beans gum. The thickening function may also be accomplished by a material that also serves as a silicone or emollient. For instance, silicone gums in excess of 10 centistokes and esters such as glycerol stearate, which have dual functionality, may be included. Suitable gelling agents may include a hydrophilic gelling agent. Hydrophilic gelling agents include carboxyvinyl polymers (carbomer), acrylic copolymers such as acrylate/alkylacrylate copolymers, polyacrylamides, polysaccharides, such as hydroxypropylcellulose, natural gums and clays, and, as lipophilic gelling agents, representative are the modified clays such as bentones, fatty acid metal salts such as aluminum stearates and hydrophobic silica, or ethylcellulose and polyethylene.

The cosmetic composition of the disclosure may also include a suitable powder, Suitable powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof.

Other adjunct components may also be incorporated into the cosmetic compositions. Such ingredients may include coloring agents, opacifiers and perfumes. Specifically, these ingredients may include cosmetically suitable additives such as deionized water, hydrolyzed glycosaminoglycan, sodium hyaluraonate, triethanolamine, propylene glycol, methylparaben, propylparaben, acrylates, C10-C20 alkyl acrylate crosspolymers, C12-C15 alkyl benzoate, panthenol, biotin, sodium chloride or sodium phosphate. Amounts of such other adjunct components may range from 0.001% or less up to 20% or more by weight of the composition.

In certain aspects, a prostaglandins based composition of the disclosure may be a hair conditioning composition, such as rinse-out hair care composition (e.g., rinses, treatments) and/or a leave-on hair product. Such hair care compositions may be based on O/W emulsions and contain water, and at least one hydrophobic ingredient (especially at least one fatty alcohol).

The amount and concentration of the prostaglandin based compound in the compositions of the disclosure may vary on several factors, such as: the pH and condition of the skin, oil content, and the interaction between the various constituents included in the composition, but should be such to be effective to at least enhance the appearance of one or more properties associated with hair follicles in a subject, such as luster, sheen, brilliance, gloss, glow, shine, patina, length, thickness, density, overall appearance of the hair, and/or the like. For instance, a cosmetic composition may includes at least 0.00001%, such as at least 0001%, for instance, at least 0.001%, for example, at least 0.01%, at least 0.03%, at least 0,1%, at least .2%, at least .3%, at least 3.5%, at least 1%, at least 2.5%, at least 5% or more, and usually not more than 10% - 20% or 30% - 50% (weight/weight) of a prostaglandin based compound as an active ingredient. The cosmetically acceptably vehicle, on the other hand, may form from 2%-5% or less to 99.9% or more, for instance, from 25% to 80%, including 35% to 60% by weight of the composition, and can, in the absence of other cosmetic adjuncts, form the balance of the composition.

In certain embodiments, a composition of the disclosure is formulated as a topical composition that releases the prostaglandin based compound all at once upon application, so as to produce a highly concentrated layer of the prostaglandin based compound on the surface of the skin, which therefore may work on the outer layer of the skin before absorption thereby. However, in certain embodiments, a composition of the disclosure may be formulated in conjunction with a controlled release mechanism, such as a microsponge, microsphere, liposome, microcapsule, polymer, gel, hydrophilic gum, and/or other colloidal drug delivery systems, so as to produce a prolonged release effect which may reduce or prevent excessive accumulation of the prostaglandin based compound within the epidermis and the dermis, so as to allow for greater penetration of the prostaglandin based compound. Because of the sustained release, the use of such controlled release mechanisms, in conjunction with the cosmetic carrier, can significantly reduce the irritation of the prostaglandin based compound without reducing its efficacy.

Accordingly, in certain embodiments, the composition may be provided in a substance or carrier that facilitates penetration and/or includes a controlled release mechanism. Examples of controlled release mechanisms include microsponges, microspheres, liposomes, microcapsules, polymers, gels, hydrophilic gums, and/or other colloidal drug delivery systems. Hence, a cosmetic composition of the disclosure may include one or more of: microsponges, microspheres (e.g., micelles), and/or liposomes.

For instance, the composition may include a controlled release mechanism such as a microsponge. Microsponges are microscopic, porous spherical sponges. Generally, microsponges are porous microspheres having a myriad of interconnected voids of particle size range 5-300µm. Depending upon the size, the total pore length may range up to 10 ft and pore volume may range up to 1 ml/g. A suitable microsponge of the disclosure has the capacity to entrap a prostaglandin based compound and/or other cosmetically suitable compound, such as an emollient, surfactant, essential oils, sunscreen and anti-infective, etc. and can be used with the prostaglandin based compound of the disclsoure as a topical carrier system. The prostaglandins based compound of the disclosure can be incorporated in to a micrsoponge and formulated into creams, lotions and powders. Other forms of microspheres may be incorporated into the present formulations of the disclosure, such as those formed from lipids, typically charged lipids, such as phospholipids.

Further, the composition may include a controlled release mechanism such as a microsphere or micelle. Micelles are comprised of surfactant molecules that are arranged so that their polar headgroups form an outer spherical shell, while the hydrophobic, hydrocarbon chains are oriented towards the center of the sphere, forming a core. Micelles form in an aqueous solution containing surfactant at a high enough concentration so that micelles naturally result. Surfactants useful for forming micelles may include, potassium laurate, sodium octane sulfonate, sodium decane sulfonate, sodium dodecane sulfonate, sodium lauryl sulfate, docusate sodium, decyltrimethylammonium bromide, dodecyltrimethylammonium bromide, tetradecyltrimethylammonium bromide, tetradecyltrimethyl-ammonium chloride, dodecylammonium chloride, polyoxyl 8 dodecyl ether, polyoxyl 12 dodecyl either, nonoxynol 10 and nonoxynol 30.

Additionally, the composition may include a controlled release mechanism such as a Liposome. Liposomes are vesicles having a lipid wall comprising a lipid bilayer. Liposomal preparations for use in the instant disclosure include cationic (positively charged), anionic (negatively charged) and neutral preparations. Cationic liposomes are readily available. For example, N-[1-2,3-dioleyloxy)propyl]-N,N,N-triethylammonium liposomes are commercially available, Anionic and neutral liposomes are commercially and readily available as well, or can be easily prepared using readily available materials. Such materials may include phosphatidyl choline, cholesterol, phosphatidyl ethanolamine, dioleoylphosphatidyl choline, dioleoylphosphatidyl glycerol, dioleoylphoshatidyl ethanolamine, among others. These materials may be mixed with N-[1-2,3-dioleyloxy)propyl]-N,N,N-triethylammonium (DOTMA) in appropriate ratios. Methods for making liposomes using these materials are well known in the art.

The cosmetic compositions of the present disclosure may also contain agents that sooth or condition the skin and hair ("conditioning agents"). One such agent is panthenol, a pro-vitamin moisturizing agent, such as Vitamin E. Panthenol is may be incorporated into the cosmetic formulations of the disclosure and may promote the penetration of the prostaglandins based compound into the skin and hair. Panthenol derivatives (e.g., ethyl panthenol) also find use in the compositions of the disclsoure as do agents such as aloe vera, pantothenic acid and its derivatives, allantoin, bisabolol, and dipotassium glycyrrhizinate. For instance, a hair conditioning agent may be a hydrolyzed wheat protein, which may be included so as to improve the hair's resilience and promote body by penetrating the hair shaft to repair damage and is provided in an amount from 5 to 25% by weight.

Other hair and skin conditioning/soothing agents may also be included in the subject compositions. For example, one or more anti-microbial agents can be included in the composition, e.g., leuconostoc/radish root ferment filtrate. If desired, a pH stabilizer such as triethanoolamine can be included in the composition, as can antioxidants, such as ascorbyl palmitate, tocopheryl acetate, L-camosine, Carotenoids, CoEnzyme Q10, Vitamin A, B, C, D, and/or E, Green Tea extract, Selenium or Zinc; or a moisturizing agent such as xodium hyaluronate; or a chelator, such as ethylenediamine, porphine, EDTA (ethylenediamine tetraacetate), DMSO, DMSO₂ (MSM), sodium phytate, DTPA (Diethylenetriaminepentaacetic acid) or NTA ACID (Nitrilotriacetic acids.

Other minor components may also be incorporated into the cosmetic compositions. These ingredients may include coloring agents, opacifiers and perfumes. Amounts of these other minor components may range anywhere from 0.00 1% or less up to 20% or more by weight of the composition.

### Preparing Compositions of the Invention

In certain embodiments, in preparing a composition of the disclosure, a prostaglandins described above may be mixed with a dermatologically compatible vehicle or carrier. The vehicle may comprise, for example, aqueous solutions, such as e.g., physiological salines, oil solutions, and/or ointments. The vehicle, furthermore may contain dermatologically compatible preservatives, such as e.g., benzalkonium chloride; surfactants like e.g., polysorbate 80; liposomes or polymers, for example, methyl cellulose, polyvinyl alcohol, polyvinyl pyrrolidone and hyaluronic acid; and/or other agents that may be used for increasing the viscosity. Furthermore, it is also possible to use soluble or insoluble drug inserts when the drug is to be administered.

Effective amounts of the prostaglandin based compound will vary depending on the compound employed, frequency of application and desired result, but will generally range from 0.0000001 to 50% by weight of the dermatological composition. Representative compositions may thus comprise from 0.001 to 50µg of the derivatives in 1 to 100µg of total dermatological composition, more preferably from 0.1 to 30 µg in 10 to 50µg of the composition. Depending on the actual formulation and prostaglandin based compounds to be used, various amounts of the drug and different dose regimens may be employed for delivery,

Accordingly, in certain aspects, a composition of the disclosure may be delivered or otherwise administered to a subject for the enhancement of the appearance of one or more properties associated with hair follicles in a subject, such as luster, sheen, brilliance, gloss, glow, shine, patina, length, thickness, density, or overall appearance of the hair. As described above the composition may be a prostaglandins based compound, such as a prostaglandin or prostamide, and may be delivered by itself or in association with a stabilizing agent, and/or with a topical cosmetic carrier. The method may include the provision of a suitably formulated composition of the disclosure and the contacting of the composition with the skin of the user. The contacting may be performed via the use of a contacting facilitator such as a brush or swab or other applicator, and the contacting may be performed by brushing, dabbing, rubbing, and/or using any other manner known in the art for contacting a composition of the disclosure with the skin and/or hair of the user.

For instance, the skift to be contacted may be the skin surrounding the eyelids, eyebrows, and/or the head of the user. In some instances, the skin to be contacted is skin that is associated with hair follicles. Accordingly, in certain embodiments, such as where the composition is formulated as an aqueous solution, an effective amount of a composition of the disclosure may be applied to the skin and/or hair by contacting a suitable applicator to the composition to be administered, so as to wet the applicator, and subsequently contacting the skin of the user with the wetted applicator. In other embodiments, such as where the composition is formulated as a topical cream, lotion, ointment, wash, rinse, or conditioner, an effective amount of the composition may be applied to the hands, a towel, swab, and/or the like, and then applied to the skin and/or hair to be contacted by rubbing. Additionally, where the composition comprises a powder, the powder may be applied by patting and/or dabbing; and where the composition is formulated as an aerosol, the contacting may be effectuated by spraying the area to be contacted with the appropriately formulated aerosol composition.

The present disclosure finds application in all mammalian species, including both human and animals, In humans, the compounds of the subject disclosure can be applied for example, to the scalp, face, beard, head, pubic area, upper lip, eyebrows, and eyelids. In animals raised for their pelts, e.g., mink, the compounds can be applied over the entire surface of the body to improve the overall pelt for commercial reasons. The process can also be used for cosmetic reasons in animals, e.g., applied to the skin of dogs and cats having bald patches due to mange or other diseases causing a degree of alopecia.

The cosmetic compositions of the invention include cosmetic compositions suited for topical and local action. The term "topical" as used herein relates to the use of a prostaglandin based compound as described herein, incorporated in a suitable cosmetic carrier, and applied at the site for exerting local action. ,4accordingly, such topical compositions including those forms in which the compound is applied externally by direct contact with the skin surface to be treated. Conventional cosmetic forms for this purpose include ointments, liniments, creams, shampoos, lotions, pastes, jellies, sprays or aerosolsand may be applied in patches or impregnated dressings depending on the part of the body to be treated. The term "ointment" embraces formulations (including creams) having oleaginous, absorption, water-soluble and emulsion-type bases, e.g., petrolatum, lanolin, polyethylene glycols, as well as mixtures of these.

For topical use on the eyelids or eyebrows, the active prostaglandin based compounds, including esters and salts, can be formulated in aqueous solutions, creams, ointments or oils exhibiting physiologically acceptable osmolarity by addition of pharmacologically acceptable buffers and salts, as described herein above. Such formulations may or may not, depending on the dispenser, contain preservatives, such as benzalkonium chloride, chlorhexidine, chlorobutanol, parahydroxybenzoic acids and phenylmercuric salts such as nitrate, chloride, acetate, and borate, or antioxidants, as well as additives like EDTA, sorbitol, boric acid etc. as additives. Furthermore, particularly aqueous solutions may contain viscosity increasing agents such as polysaccharides, e.g., methylcellulose, mucopolysaccharides, e.g., hyaluronic acid and chondroitin sulfate, or polyalcohol, e.g., polyvinylalcohol. Various slow releasing gels and matrices may also be employed as well as soluble and insoluble ocular inserts, for instance, based on substances forming in-situ gels.

Depending on the actual formulation and prostaglandin based compound to be used, various amounts of the compound and different dose regimens may be employed. In use, a quantity of the composition, for example from 0.0001 ml to 100 ml, from 0.001 ml to 113 ml, from 0.01 ml to I ml, such as from 0.1 ml is applied to a site of interest (i.e., skin or hair of the eyelash, eyebrow, and/or scalp) from a suitable container or applicator and, if necessary, it may then spread over and/or rubbed into the site using a suitable device or the hand or finger. For instance, the daily amount of the prostaglandin based compound for contact with the eyelid may be 0.1 ng to 100 mg per eyelid, more preferably 1 ng to 100 µg per eyelid. For example, in some embodiments, a topical application of a composition of the disclosure may involve the application of a dosage or 1.5 µg/ml per area to be contacted, e.g., eyelid, per day. However, this application may be increased to twice or three times daily as desired.

The duration of the administration may vary dependent on the desired effects and in certain embodiments should be maintained to ensure the maintenance of said desired effects. For instance, the duration may be for 1, 2, 4, 6, 8, 10, 12, or more weeks or more. For example, the duration may be daily or twice daily for 1, 2, 4, 6, 8, 10, 12 or more months. In some embodiments, a regime may be set up wherein the application occurs once or twice daily for the first month, two, or three, or more, and then a maintenance period begins wherein a once, twice, or three times a week application occurs. In this manner an individual dosage regime may be set up so as to maximize the users desired results.

The product may be specifically formulated for use as a conditioner for a specific area, e.g. the eyelashes, eyebrows, the face, the hair, or the scalp. To achieve the daily amount of conditioning depending on the formulation, the prostaglandin based compound may be administered once or several times daily with or without antioxidants.

In certain aspects, the disclosure is directed to a kit wherein the kit may include: a composition of the disclosure, where the composition is contained within a suitable container, such as a jar, tube, vial, aerosol can, or the like; directions for using the composition: and/or suitable packaging for containing one or more of the composition, container, and/or directions. In certain embodiments, the directions for use may be included on or in the packaging. For instance, in certain embodiments, the directions may be written or printed out on a separate piece of paper, or may be digitally encoded, for instance, on a CD or DVD Rom device. In certain embodiments, the directions for use may simply be a reference to where a more detailed set of instructions for use may be obtained, for instance, an on-line web address.

An exemplary composition of the disclosure will be illustrated in the following non-limiting examples,

Example 1. Pre-blend Composition

| **%W/W** | **Ingredients:** | **INCI:** |
|---|---|---|
| 93.80% | Cetiol AB (Phase A) | C12-15 Alkyl Benzoate |
| 4.20% | Prostaglandin Based Compound (Phase B) | 17-phenyl trinor Prostaglandins E2 serinol amide |
| 2.00% | SDA 40B (Phase C) | Ethanol |

Pre-blend ingredient list:

The Cetiol AB (including C12-15 alkyl benzoate) may be present in QS.

Prostaglandin E₂ or derivative thereof may be present in an amount that ranges from 0.001% or less to 15% or more, such as from 0.01% to 12%, including 0.1% to 10%, for instance, from 1% to 5%, including 4-4.5%.

The SDA40B (e.g., Ethanol) may be present in an amount from 0.001% or less to 15% or more, such as from 0.01% to 10%, including 0.1% to 9%, for instance, from 1% to 8%.

This blend may then be mixed in the final formulation at, for example, 0.34%.

Exemplary method for preparing a pre-blend:
1. In a stall container, weigh Prostaglandins Based Compound of the disclosure.
2. Add Ethanol.
3. Add C12-15 Alkyl Benzoate and mix for 10 minutes, solution should be clear.
4. Mix and add to eyelash luster enhancing composition.

Example 2. Direct-blend Composition

| **% W/W** | **Ingredients:** | **INCI:** |
|---|---|---|
| 4.20% | Prostaglandin Based Compound (Phase B) | 17-phenyl trinor Prostaglandin E2 serinol amide |

Direct-blend ingredient list:

In certain embodiments, the Prostaglandin E₂ or derivative thereof may be present in an amount that ranges from 0.001% or less to 15% or more, such as from 0.01% to 12%, including 0.1% to 10%, for instance, from 1% to 5%, including 4-4.5%. This direct-blend may be mixed directly into the final formulation, e.g., without prior blending with C12-15 Alkyl Benzoate and/or ethanol, at, for example, 0.30%.

Exemplary method for preparing a pre-blend:
1. In a small container, weigh Prostaglandins Based Compound of the disclosure.
2. Add to eyelash fluster enhancing composition directly.

Example 3. Eyelash luster enhancing composition

| **% W/W** | **Ingredients:** | **INCI:** |
|---|---|---|
| 85.53% | Water | Water |
| 0535% | Cavamax | Cyclodextrin |
| 0.60% | Pemulen | Acrylates/C10-30 Alkyl Acrylate Crosspo!ymer |
| 0.10% | Panthenol | Panthenol |
| 0.03% | Ritaloe 200M | Aloe Barbadensis Leaf |

| **%W/W** | **Ingredients:** | **INCI:** |
|---|---|---|
| | | Juice |
| 2.00% | AMS Leucidal Liquid | Leuconostoc/Radish Root Ferment Filtrate |
| 0.80% | TEA 99% | Trietholamine |
| 0.25% | Liposome ACE | Water (and) Phospholipids (and) Retinyl Palmitate (and) Ascorbyl Palmitate (and) Tocopheryl Acetate |
| 10.00% | Actiglide | Water (and) Sodium Hyaluronate (and) Hydrolyzed Glycosaminoglycans |
| 0.3%- 0.34% | Eyelash Pre- or Direct- blend | |

Exemplary method for preparing eyelash luster enhancing composition:
1. In a small container add Phase B.
2. Add Phase C.
3. Add Phase A and mix for 10 minutes, solution should be clear.
   Finished formula
   A-Water -QS
   A-Cavamax -0-5%
   A-Pemulen -0-1%
   A-Panthenol -0-1%
   A-Ritaloe 200M -0-1%,
   B-AMS Leucidal Liquid -0-2.5%
   C-TEA 99% -0-1%
   D-Liposome ACE -0-1%
   D-Actiglide -0-20%
   D-Eyelash Preblend -0-5%

Specifically, charge water into main and begin propeller mixing. Sift in each ingredient of Phase A and mix until completely dispersed. Switch to sweep mixing. Add Phase B. Add Phase C. And Add Phase D.

Final pH of the formulation may be adjusted so to be in a range of pH-5.0-6.5.

Example 4. Evaluation of Composition:

A prostaglandin based composition of the disclosure, such as that set forth above in Example 3, was studied so as to evaluate the ability of the composition to enhance the appearance of one or more properties associated with hair follicles in a subject, such as luster, sheen, brilliance, gloss, glow, shine, patina, length, thickness, density, or overall appearance of the hair, as well as to evaluate the potential for irritation. Thirty-five female subjects were tested. Each subject was provided with the prostaglandin based composition and given instructions as to its use. Specifically, test subjects were instructed to apply the prostaglandin based composition to the root of the upper lashes of both eyes, following makeup removal, once nightly tor twelve weeks.

Computerized consumer perception questionnaires were completed by each subject at the end of Weeks: 2, 4, 6, 8, and 12. A questionnaire setting forth several inquiries with regard to the efficacy of the prostaglandin based composition following use. The questionnaires were designed to provide an efficient and accurate method of determining the test subject's response proportions and to assess the consensus opinion of the clinical study population. Questionnaire responses were analyzed by Z-tests. The Z-tests were used to determine the statistically significant differences in the proportions of test subjects responding positively or negatively to each test parameter questioned and offering a range of responses. The proportions of subjects choosing the neutral response were split equally and added to the response proportion of the top and bottom choices. The split proportions were compared by the calculation of the Z-Score so as to determine the statistically significant differences. Statistical significance was determined to exist for Z-Scores greater than or equal to the absolute value of 1.96 at the 95% confidence level.

Subjective and objective ocular examinations were performed at Weeks: 0, 6, and 12. Specifically, the ocular status of the test subjects was assessed with regard to several examination parameters including: the degree of lacrimation; redness, scaling, swelling, and meibomian secretions of the eyelids; follicular and/or papillary reaction of the palpebral conjunctivae; evidence of inflammation or abnormalities of the bulbar conjunctivae; evidence of neovascularization, edema, infiltrates, capacities, and/or epithelial defects of the cornea; evidence of scratches and deposits on contact lenses; and intraocular pressure.

Of the 35 subjects tested, 77.1% acknowledged that a prostaglandin based composition of the disclosure enhanced the condition of the lashes after 4 weeks of use. 70.0% acknowledged that the composition made the lashes softer after 2 weeks of use and 80.0% acknowledged this after 6 weeks of use. 67.1% acknowledged that the composition made the lashes feel silkier after 2 weeks of use and 78.6% acknowledged this after 6 weeks of use. 68.6% acknowledged that the composition made the lashes less sparse after 4 weeks of use and 68.6% acknowledged this after 6 weeks of use. 61.4% acknowledged that a prostaglandin based composition of the disclosure enhanced the appearance of the length of the lashes after 4 and 6 weeks of use. 61.4% acknowledged that a prostaglandin based composition of the disclosure enhanced the appearance of the length of the lashes after 4 and 6 weeks of use. 67.1% acknowledged that the composition enhanced the appearance of the thickness of the lashes after 4 weeks of use. 61.4% acknowledged that the composition enhanced the appearance of the fullness of the lashes after 4 and 6 weeks of use. 67.1% acknowledged that the composition made the lashes healthier looking after 4 weeks of use and 68.6% acknowledged this after 6 weeks of use. 67.1% acknowledged that the composition enhanced the lashes natural look after 4 weeks of use and 74.3% acknowledged this after 6 weeks of use. Additionally, 68.6% acknowledged that the prostaglandin based composition of the disclosure enhanced the overall appearance of the lashes after 4 weeks of use and 70.0% acknowledged this after 6 weeks of use.

## Claims

1. A topical composition for application to mammalian skin, eyebrow or eyelash comprising
a prostaglandin based compound; and
optionally, a biologically acceptable carrier,
wherein said prostaglandin based compound is 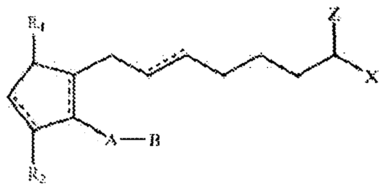 wherein the dashed bonds represent a single or double bond which can be in the cis or trans configuration,
A is an alkylene or alkenylene radical having from two to six carbon atoms, which radical may be interrupted by one or more oxa radicals and substituted with one or more hydroxy, oxo, alkyloxy or akylcarboxy groups wherein said alkyl radical comprises from one to six carbon atoms;
B is a cycloalkyl radical having from three to seven carbon atoms, or an aryl radical, selected from the group consisting of hydrocarbyl aryl and heteroaryl radicals having from four to ten carbon atoms wherein the heteroatom is selected from the group consisting of nitrogen, oxygen and sulfur atoms;
X is either -N(H)CH(CH₂OH)₂ or -OCH(CH₂OH)₂;
Z is =O;
one of R₁ and R₂ is =O, -OH or a -O(CO)R₃ group, and the other one is -OH or - O(CO)R₃, or R₁ is =O and R₂ is H, wherein R₃ is a saturated or unsaturated acyclic hydrocarbon group having from 1 to 20 carbon atoms, or -(CH₂)mR₄ wherein m is 0 or an integer of from 1 to 10, and R₄ is cycloalkyl radical, having from three to seven carbon atoms, or a hydrocarbyl aryl or heteroaryl radical, or a pharmacologically acceptably acid addition salt thereof.

2. The composition of claim 1, wherein said prostaglandin based compound is either [(2-hydroxy-1-hydroxymethyl)ethyl]-9-oxo-11,15-dihydroxy-17-phenyl-18, 19, 20-trinor-prosta-5Z, 13E-dien-1-oate or 17-phenyl trinor prostaglandin E2 serinol amide.

3. The composition of claim 1, further comprising a stabilizing agent, wherein preferably said stabilizing agent is a Cyclodextrin or dextran, dextrin, or pullulan.

4. The composition of claim 3, wherein said cyclodextrin is a selected from the group consisting of α-cyclodextrin, β-cyclodextrin, a γ-cyclodextrin, 2-hydroxypropyl-alpha-cyclodextrin, 2-hydroxypropyl-beta-cyclodextrin, sulfobutyl ether-beta-cyclodextrin, and 2,6-dimethyl-beta cyclodextrin.

5. A liposome comprising the composition of claim 1.

6. The liposome of claim 5, wherein said liposome comprises a phospholipid wherein preferably, said Liposome further comprises retinyl palmitate, ascorbyl palmitate, or tocopheryl acetate.

7. The composition of claim 1, wherein said composition further comprises an alkyl benzoate.

8. The composition of claim 1, wherein said composition further comprises a preservative .

9. The composition of claim 1, wherein said composition further comprises a moisturizing agent.

10. A method for the production of a composition according to claim 7, comprising contacting said prostaglandin based compound and alkyl benzoate with an alcohol prior to formulation of the composition or providing said prostaglandin based compound admixed with said alkyl benzoate wherein preferably said alkyl benzoate is C₁₂₋₁₅ alkyl benzoate.

11. A method of enhancing the appearance of at least one property of a subject's hair selected from the group consisting of: luster, sheen, brilliance, gloss, glow, shine, patina, length, thickness, and density; the method comprising contacting a portion of skin of a subject with the composition of claim 1, wherein said skin is associated with hair follicles and said contacting results in the enhancement of the appearance of the at least one property of the hair of the subject.

12. The method of claim 11, wherein the skin to be contacted is selected from the group consisting of skin associated with an eyelash, skin associated with an eyebrow, skin associated with a scalp, and skin associated with a face of the subject.

13. The method of claim 11, wherein said composition is formulated as an aqueous solution, an ointment, a liniments, a cream, a lotion, a paste, jelly, a shampoo, a rinse, a conditioner, an aerosol, or a spray.

14. The method of claim 11, wherein said contacting is performed at least once a day.

15. The method of claim 14, wherein said contacting is performed for at least 3 months.

## Patentansprüche

1. Topische Zusammensetzung zum Aufbringen auf die Haut, die Augenbrauen oder die Augenwimpern eines Säugers, umfassend
eine Verbindung auf Prostaglandinbasis und
gegebenenfalls einen biologisch verträglichen Träger,
wobei die Verbindung auf Prostaglandinbasis 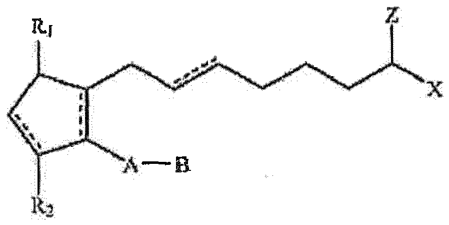 ist, worin die gestrichelten Bindungen eine Einfach- oder Doppelbindung, die in der cis- oder trans-Konfiguration vorliegen kann, darstellen,
A ein Alkylen- oder Alkenylenrest mit zwei bis sechs Kohlenstoffatomen ist, wobei der Rest durch einen oder mehrere Oxarest(e) unterbrochen und mit einer oder mehreren Hydroxy-, Oxo-, Alkyloxy- oder Alkylcarboxygruppe(n) substituiert sein kann, wobei der Alkylrest ein bis sechs Kohlenstoffatom(e) umfasst,
B ein Cycloalkylrest mit drei bis sieben Kohlenstoffatomen oder ein Arylrest ist, der aus der Gruppe, bestehend aus Hydrocarbylaryl- und Heteroarylresten mit vier bis zehn Kohlenstoffatomen, ausgewählt ist, wobei das Heteroatom aus der Gruppe, bestehend aus Stickstoff-, Sauerstoff- und Schwefelatomen ausgewählt ist,
X entweder -N(H)CH(CH₂OH)₂ oder -OCH(CH₂OH)₂ ist,
Z =O ist,
einer von R₁ und R₂ =O, -OH oder eine -O(CO)R₃-Gruppe ist und der andere -OH oder -O(CO)R₃ ist, oder R₁ =O ist und R₂ H ist, worin R₃ eine gesättigte oder ungesättigte acyclische Kohlenwasserstoffgruppe mit 1 bis 20 Kohlenstoffatomen oder -(CH₂)mR₄ ist, worin m 0 oder eine ganze Zahl von 1 bis 10 ist, und R₄ ein Cycloalkylrest mit drei bis sieben Kohlenstoffatomen oder ein Hydrocarbylaryl- oder Heteroarylrest ist,
oder ein pharmazeutisch verträgliches Säureadditionssalz davon.

2. Zusammensetzung nach Anspruch 1, bei der die Verbindung auf Prostaglandinbasis entweder [(2-Hydroxy-1-hydroxymethyl)ethyl]-9-oxo- 1,15-dihydroxy-17-phenyl-18,19,20-trinor-prosta-5Z,13E-dien-1-oat oder 17-Phenyltrinorprostaglandin-E2-serinolamid ist.

3. Zusammensetzung nach Anspruch 1, die ferner ein Stabilisierungsmittel umfasst, wobei das Stabilisierungsmittel vorzugsweise ein Cyclodextrin oder Dextran, Dextrin oder Pullulan ist.

4. Zusammensetzung nach Anspruch 3, bei der das Cyclodextrin aus der Gruppe, bestehend aus α-Cyclodextrin, β-Cyclodextrin, einem γ-Cyclodextrin, 2-Hydroxypropyl-alpha-cyclodextrin, 2-Hydroxypropyl-beta-cyclodextrin, Sulfobutylether-beta-cyclodextrin und 2,6-Dimethyl-beta-cyclodextrin, ausgewählt ist.

5. Liposom, das die Zusammensetzung von Anspruch 1 umfasst.

6. Liposom nach Anspruch 5, wobei das Liposom ein Phospholipid umfasst, wobei das Liposom vorzugsweise ferner Retinylpalmitat, Ascorbylpalmitat oder Tocopherylacetat umfasst.

7. Zusammensetzung nach Anspruch 1, wobei die Zusammensetzung ferner ein Alkylbenzoat umfasst.

8. Zusammensetzung nach Anspruch 1, wobei die Zusammensetzung ferner ein Konsenrierungsmittel umfasst.

9. Zusammensetzung nach Anspruch 1, wobei die Zusammensetzung ferner ein Feuchthaltemittel umfasst.

10. Verfahren zur Herstellung einer Zusammensetzung nach Anspruch 7, umfassend das Inkontaktbringen der Verbindung auf Prostaglandinbasis und Alkylbenzoat mit einem Alkohol vor der Formulierung der Zusammensetzung oder das Bereitstellen der Verbindung auf Prostaglandinbasis gemischt mit dem Alkylbenzoat, wobei das Alkylbenzoat vorzugsweise ein C₁₂₋₁₅-Alkylbenzoat ist.

11. Verfahren zur Verbesserung des Auftretens von mindestens einer Eigenschaft des Haares eines Lebewesens, ausgewählt aus der Gruppe, bestehend aus schimmerndem Glanz, Schimmer, Brillianz, Glanz, Leuchten, Strahlen, Patina, Länge, Dicke und Dichte, wobei das Verfahren das Inkontaktbringen eines Abschnitts der Haut eines Lebewesens mit der Zusammensetzung von Anspruch 1 umfasst, wobei die Haut mit Haarfollikeln verbunden ist und das Inkontaktbringen zu einer Verbesserung des Auftretens von mindestens einer Eigenschaft des Haares des Lebewesens führt.

12. Verfahren nach Anspruch 11, wobei die Haut, die in Kontakt gebracht werden soll, aus der Gruppe, bestehend aus Haut, die mit einer Augenwimper verbunden ist, Haut, die mit einer Augenbraue verbunden ist, Kopfhaut und Gesichtshaut des Lebewesens, ausgewählt ist.

13. Verfahren nach Anspruch 11, wobei die Zusammensetzung als eine wässrige Lösung, eine Salbe, ein Einreibemittel, eine Creme, eine Lotion, eine Paste, Gel, ein Shampoo, eine Spülung, ein Konditionierer, ein Aerosol oder ein Spray formuliert ist.

14. Verfahren nach Anspruch 11, wobei das Inkontaktbringen mindestens einmal täglich durchgeführt wird.

15. Verfahren nach Anspruch 14, wobei das Inkontaktbringen für mindestens drei Monate durchgeführt wird.

## Revendications

1. Composition topique pour application à la peau, les sourcils ou les cils d'un mammifère, comprenant un composé à base de prostaglandine ; et
éventuellement un véhicule biologiquement acceptable, dans laquelle ledit composé à base de prostaglandine est 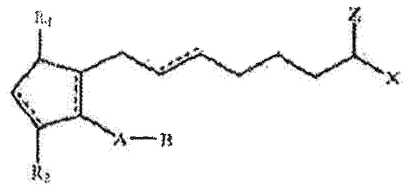 où les liaisons en tirets représentent une liaison simple ou double qui peut être en configuration cis ou trans,
A est un radical alkylène ou alcénylène ayant de deux à six atomes de carbone, lequel radical peut être interrompu par un ou plusieurs radicaux oxa et substitué par un ou plusieurs groupes hydroxy, oxo, alkyloxy ou alkylcarboxy où ledit radical alkyle comprend d'un à six atomes de carbone ;
B est un radical cycloalkyle ayant de trois à sept atomes de carbone, ou un radical aryle, choisi dans l'ensemble constitué par les radicaux hydrocarbyle aryle et hétéroaryle ayant de quatre à dix atomes de carbone où l'hétéroatome est choisi dans l'ensemble constitué par les atomes d'azote, d'oxygène et de soufre ;
X est soit -N(H)CH(CH₂OH)₂ soit -OCH(CH₂OH)₂ ;
Z est =O ;
l'un de R₁ et R₂ est =O, -OH ou un groupe -O (CO) R₃, et l'autre est -OH ou -O(CO)R₃, ou bien R₁ est =O et R₂ est H, où R₃ est un groupe hydrocarboné acyclique saturé ou insaturé ayant de 1 à 20 atomes de carbone, ou -(CH₂)ₘR₄ où m vaut 0 ou est un entier de 1 à 10, et R₄ est un radical cycloalkyle, ayant de trois à sept atomes de carbone, ou un radical hydrocarbyle aryle ou hétéroaryle,
ou un sel d'addition d'acide pharmacologiquement acceptable de celui-ci.

2. Composition selon la revendication 1, dans laquelle ledit composé à base de prostaglandine est soit le [(2-hydroxy-1-hydroxyméthyl)éthyl]-9-oxo-11,15-dihydroxy-17-phényl-18,19,20-trinor-prosta-5Z,13E-dién-1-oate soit le sérinol-amide de 17-phényl-trinor-prostaglandine E2.

3. Composition selon la revendication 1, comprenant en outre un agent stabilisant, dans laquelle de préférence ledit agent stabilisant est une cyclodextrine ou un dextrane, la dextrine, ou le pullulane.

4. Composition selon la revendication 3, dans laquelle ladite cyclodextrine est choisie dans l'ensemble constitué par l'α-cyclodextrine, la β-cyclodextrine, une γ-cyclodextrine, la 2-hydroxypropyl-α-cyclodextrine, la 2-hydroxypropyl-β-cyclodextrine, l'éther sulfobutylique de β-cyclodextrine, et la 2,6-diméthyl-β-cyclodextrine.

5. Liposome comprenant la composition de la revendication 1.

6. Liposome selon la revendication 5, lequel liposome comprend un phospholipide, et de préférence lequel liposome comprend en outre du palmitate de rétinyle, du palmitate d'ascorbyle, ou de l'acétate de tocophéryle.

7. Composition selon la revendication 1, laquelle composition comprend en outre un benzoate d'alkyle.

8. Composition selon la revendication 1, laquelle composition comprend en outre un conservateur.

9. Composition selon la revendication 1, laquelle composition comprend en outre un agent hydratant.

10. Procédé pour la production d'une composition selon la revendication 7, comprenant la mise en contact dudit composé à base de prostaglandine et de benzoate d'alkyle avec un alcool avant formulation de la composition, ou le fait de disposer ledit composé à base de prostaglandine en mélange avec ledit benzoate d'alkyle, dans lequel, de préférence, ledit benzoate d'alkyle est un benzoate d'alkyle en C₁₂ à C₁₅ .

11. Procédé pour amplifier l'apparition d'au moins une propriété des poils d'un sujet, choisie dans l'ensemble constitué par : le lustre, le reflet, la brillance, le brillant, l'éclat, la patine, la longueur, l'épaisseur, et la densité ; lequel procédé comprend la mise en contact d'une partie de la peau d'un sujet avec la composition de la revendication 1, dans lequel ladite peau est associée à des follicules pileux et ladite mise en contact a pour résultat une amplification de l'apparition de l'au moins une propriété des cheveux du sujet.

12. Procédé selon la revendication 11, dans lequel la peau devant être mise en contact est choisie dans l'ensemble constitué par la peau associée aux cils, la peau associée aux sourcils, la peau associée au cuir chevelu, et la peau associée au visage du sujet.

13. Procédé selon la revendication 11, dans lequel ladite composition est formulée sous la forme d'une solution aqueuse, d'une pommade, d'un liniment, d'une crème, d'une lotion, d'une pâte, d'une gelée, d'un shampooing, d'un produit à rincer, d'un après-shampooing, d'un aérosol, ou d'un spray.

14. Procédé selon la revendication 11, dans lequel ladite mise en contact est effectuée au moins une fois par jour.

15. Procédé selon la revendication 14, dans lequel ladite mise en contact est effectuée pendant au moins 3 mois.
